# EUROPEAN PATENT SPECIFICATION

(11) **EP 3 698 744 B1**
(45) Date of publication and mention of the grant of the patent: **02.03.2022**
(21) Application number: 19863986.6
(22) Date of filing: 17.01.2019
(51) Int. Cl.: A61B 17/86

(54) **ORTHOPEDIC IMPLANT SCREW**
ORTHOPÄDISCHE IMPLANTATSCHRAUBE
VIS D'IMPLANT ORTHOPÉDIQUE

(30) Priority: 18.12.2018 CN 201811555263
(43) Date of publication of application: 26.08.2020
(73) Proprietor: Beijing AK Medical Co., Ltd., 102200, Beijing (CN)
(72) Inventor: WANG, Caimei, Beijing 102200 (CN)
(74) Representative: Nederlandsch Octrooibureau
(86) International application number: PCT/CN2019/072111
(87) International publication number: WO 2020/124722

(56) References cited:
- CN-A- 101 146 486
- CN-A- 101 932 285
- CN-A- 102 596 065
- CN-A- 103 402 448
- FR-B1- 2 827 150
- US-A1- 2008 275 510
- US-A1- 2009 192 551
- US-A1- 2009 312 800
- US-A1- 2012 010 662
- US-A1- 2013 123 848

## Description

### Technical Field

The disclosure relates to a technical field of medical instruments, and in particular to a structural design of a combined type self-stabilizing orthopedic implanted screw. The orthopedic implanted screw has bone integration and self-locking functions, thereby effectively preventing the orthopedic implanted screw from dropping out of a bone.

### Background

As a common orthopedic implant applied to fixation, a bone screw is generally applied to fixation of internal fracture or dislocation. During use, the bone screw is directly screwed into two different bone blocks or is directly screwed into fixing bone plates and other implants, to achieve fixation of a bone and facilitate healing of an affected area.

However, a screw implanted into the bone easily gets loose and even slightly screws out from the bone step by step after working in presence of a complicated stress for a long time in clinic practice, accordingly it is not only unfavorable to rapid growth of bones, and stable growth of bones and healing of the bone but also may probably cause secondary damage of the affected area, and consequently living quality of a patient is influenced seriously.

D1 (US2009/0192551A1) discloses a facet fixation prosthesis. A facet screw system for surgical implantation into bone tissue having a shaft, a compression member and a washer. The shaft includes a shaft having a bone engaging portion and a compression member engaging portion. The compression member includes a spherical head portion having a recess for engaging with a driving instrument, and an elongated coupling portion having internal threads for coupling with the compression member engaging portion of the shaft and a washer coupled to the spherical head portion of the compression member and having a plurality of bone engaging protrusions. The washer is adapted to be polyaxially rotatable with respect to the compression member.

D2 (US2012/0010662A1) discloses a pedicularfacet fusion screw with plate. Spinal implants and methods for spinal stabilization and/or fusion are provided. Exemplary implants described herein can be configured for delivery to a facet joint to stabilize and/or fuse the facet joint, and can optionally be anchored within the pedicle for added fixation. The implant can optionally include a fusion-promoting bioactive material thereby providing a single device capable of spinal stabilization and/or fusion. Furthermore, a method of placing such an implant within a facet joint is provided.

D3 (US2013/0123848A1) discloses a facet fixation systems. Facet fixation implants include a cap member with a circular base portion and an eccentric raised portion. The raised portion includes first and second lobes and is smoothly contoured to provide an unobtrusive profile when implanted. Beveled teeth project from the implant to provide fixation and compression across the facet joint. The cap member includes an offset aperture which receives a fastener. Tools, guiding instruments and methods for implantation of the implants are disclosed.

D4 (US2009/0312800A1) discloses a system and method for facet fixation. A spinal fixation element includes a screw and a washer. The screw comprises an elongated body of a first diameter and the body includes a threaded portion at a distal end and a semispherical head of a second diameter at a proximal end. The second diameter is larger than the first diameter. The washer comprises a semispherical through opening of a third diameter at the top, of a fourth diameter in the middle and of a fifth diameter at the bottom. The third diameter is slightly smaller than the second diameter, the fourth diameter is slightly larger than the second diameter and the fifth diameter is smaller than the second diameter. The washer surrounds the semispherical head, is non-removably attached to the semispherical head and is rotatable and positionable at an angle relative to the elongated body.

### Summary

Embodiments of the disclosure are to provide an orthopedic implanted screw, to solve the problem that a bone screw implanted into a bone in the conventional art may easily get loose and even slightly screw out from the bone step by step after working in presence of a complicated stress for a long time, accordingly it is not only unfavorable to quick and stable growth and healing of the bone but may probably cause secondary damage of an affected area, and consequently living quality of a patient may be influenced seriously.

In order to achieve the abovementioned objective, the disclosure provides an orthopedic implanted screw, for implantation into a bone. The orthopedic implanted screw includes a fixed seat and a screw body. The fixed seat includes a seat body, a deformation limiting portion and a taper portion. Herein, the seat body is provided with a limiting cavity, a top avoidance opening and a bottom avoidance opening communicating with the limiting cavity, the deformation limiting portion is configured on an inner wall surface of the seat body, protrudes toward an inner part of the limiting cavity and is positioned at the top avoidance opening, the taper portion is configured on an outer wall surface of the seat body in a protruding mode. The screw body is detachably mounted on the fixed seat, and the screw body drills into a bone in a rotating mode. In addition, one part of the screw body is limited in the limiting cavity by the inner wall surface of the seat body and the deformation limiting portion, another part of the screw body drills into the bone after passing through the bottom avoidance opening, and the screw body positioned in the limiting cavity abuts against the inner wall surface of the seat body so that the taper portion drills into the bone, to accordingly prevent the screw body from dropping out of the bone, the orthopedic implanted screw further comprises a pad , the pad is provided on an outer wall surface of the fixed seat, the taper portion passes through the pad, and an interior of the pad is provided with a plurality of micropore structures.

In some embodiments, the screw body may include a screw tip, a screw trunk and a screw cap that are connected in sequence. Herein, the screw tip and the screw trunk are provide with a threaded structure, or, the screw tip is provide with a threaded structure, or, the screw trunk is provide with a threaded structure, the screw tip and the screw trunk drill into the bone through the threaded structure, the screw cap is limited in the limiting cavity by the inner wall surface of the seat body and the deformation limiting portion, and the screw cap is configured to abut against the inner wall surface of the seat body.

In some embodiments, part of a surface of the inner wall surface of the seat body and part of a surface of an outer surface of the screw cap are curved surfaces fitting with each other compatibly.

In some embodiments, a surface part of the inner wall surface of the seat body fitting the outer surface of the screw cap is part of a surface of a spherical surface.

In some embodiments, the taper portion extends along an axial direction of the seat body, or the taper portion inclines toward a side opposite to a direction in which the threaded structure 24 is rotated into an inside of the bone.

In some embodiments, there are multiple taper portions, and a plurality of taper portions are provided around a circumferential direction of the seat body at intervals.

In some embodiments, the deformation limiting portion is an elastic buckle having deformation function. There are multiple elastic buckles, and multiple elastic buckles are configured around a circumferential direction of the top avoidance opening at intervals.

In some embodiments, the pad and the fixed seat are formed integrally through 3D printing.

In some embodiments, the orthopedic implanted screw comprises a screwing counter bore and a clamping slot provided on an upper surface of the screw cap , and the clamping slot is connected with the deformation limiting portion compatibly in a clamping mode.

With the adoption of the technical solution of the disclosure, the screw body is detachably mounted on the fixed seat, in this way the screw body may pass through the top avoidance opening, the limiting cavity and the bottom avoidance opening of the seat body in sequence when using the orthopedic implanted screw, and drill into the bone in a screwing mode. When the screw body drills into the bone for a certain depth, the taper portion of the fixed seat may contact with a surface of the bone. At the moment, the screw body is continued to be screwed. When the screw body continues to drill into the bone, one end of the screw body far away from the bone may abut against the deformation limiting portion of the fixed seat, the deformation limiting portion may be deformed until the one end of the screw body far away from the bone completely gets over the deformation limiting portion, the deformation limiting portion may restore an original shape, to stop an end surface of the one end of the screw body far away from the bone. Then the screw body is continued to be screwed, and the screw body positioned in the limiting cavity abuts against the inner wall surface of the seat body so that the taper portion drills into the bone. In this way, a limitation relation may be formed between the fixed seat and the bone. Meanwhile, the fixed seat further plays a role of limitation to the screw body, thereby preventing the screw body from dropping out of the bone accordingly. Stability of the screw body implanted into the bone is ensured, it is not only favorable to quick growth and stable growth and healing of the bone but prevents the affected area from secondary damage, and consequently living quality of the patient may be reliably improved.

### Brief Description of the Drawings

The accompanying drawings described herein are used to provide a further understanding of the disclosure, and constitute a part of the present application, and the exemplary embodiments of the disclosure and the description thereof are used to explain the disclosure, but do not constitute improper limitations to the disclosure. In the drawings:
Fig. 1 shows an assembling structure diagram of an orthopedic implanted screw in accordance with an optional embodiment of the disclosure.
Fig. 2 shows a front view of an orthopedic implanted screw in Fig. 1.
Fig. 3 shows a section view of an orthopedic implanted screw in Fig. 2.
Fig. 4 shows a section view of an assembling structure of a fixed seat and a pad of an orthopedic implanted screw in Fig. 1.
Fig. 5 shows a structure diagram of a fixed seat of an orthopedic implanted screw in Fig. 1.
Fig. 6 shows a front view of a fixed seat in Fig. 5.
Fig. 7 shows a section view of a fixed seat in Fig. 6.
Fig. 8 shows a section view of a pad of an orthopedic implanted screw in Fig. 1.
Fig. 9 shows a structure diagram of a screw body of an orthopedic implanted screw in Fig. 1.

Herein, the abovementioned drawings may include reference numbers below:
10: Fixed seat; 11: Seat body; 111: Limiting cavity; 112: Top avoiding opening; 113: Bottom avoiding opening; 12: Deformation limiting portion; 13: Taper portion; 20: Screw body; 21: Screw tip; 22: Screw trunk; 23: Screw cap; 231: Screwing counter bore; 232: Clamping slot; 24: Threaded structure; 30: Pad; 31: Micropore structure.

### Detailed Description of the Embodiments

The technical solutions in the embodiments of the disclosure will be clearly and completely described below in combination with the drawings in the embodiments of the disclosure. It is apparent that the described embodiments are not all embodiments but part of embodiments of the disclosure. The description of at least one exemplary embodiment below is illustrative only in fact, and is never intended to limit the disclosure and its application or use. All other embodiments obtained by those of ordinary skilled in the art on the basis of the embodiments in the disclosure without creative work shall fall within the scope of protection of the disclosure.

In order to solve the problem that a bone screw implanted into a bone in the conventional art may easily get loose and even slightly screw out from the bone step by step after working in presence of a complicated stress for a long time, accordingly it is not only unfavorable to quick growth and stable growth and healing of the bone but may probably cause secondary damage of an affected area, and consequently living quality of a patient may be influenced seriously, the disclosure provides an orthopedic implanted screw.

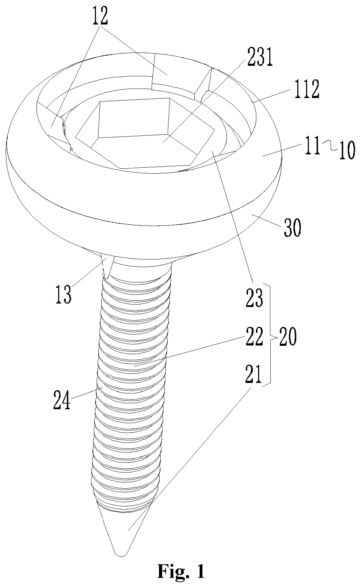

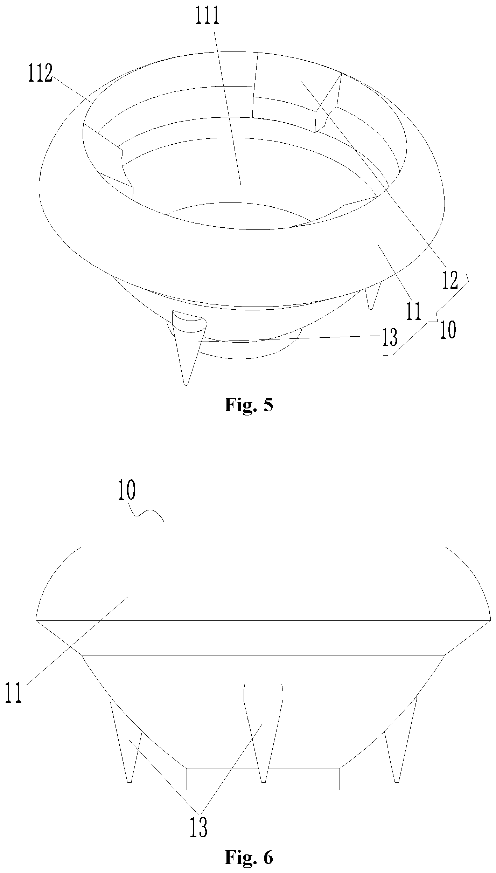

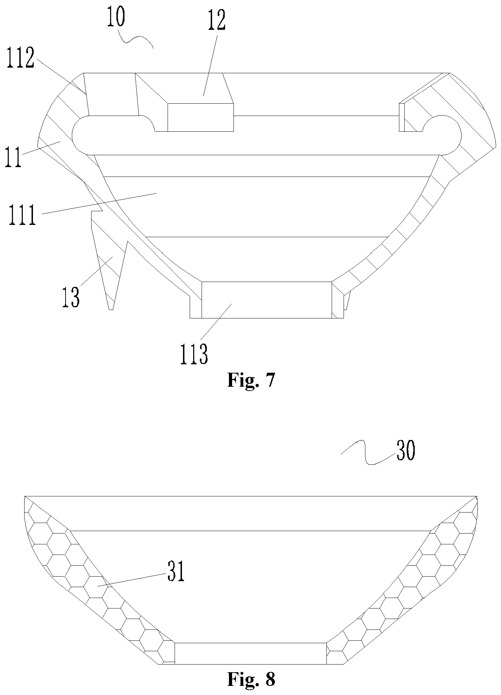

As shown in Figs. 1-9, the orthopedic implanted screw for implanting into the bone includes a fixed seat 10 and a screw body 20. The fixed seat 10 includes a seat body 11, a deformation limiting portion 12 and a taper portion 13 Herein, the seat body 11 is provided with a limiting cavity 111 and a top avoiding opening 112 and a bottom avoiding opening 113 communicating with the limiting cavity 111, the deformation limiting portion 12 is provided on an inner wall surface of the seat body 11, protrudes toward an inside of the limiting cavity 111 and is provided at the top avoiding opening 112, the taper portion 13 is convexly provided on an outer wall surface of the seat body 11. The screw body 20 is detachably mounted on the fixed seat 10. The screw body 20 being configured to drill into the bone in a rotating mode. In addition, a part of the screw body 20 is limited in the limiting cavity 111 by the inner wall surface of the seat body 11 and the deformation limiting portion 12, another part of the screw body 20 drilling into the bone after passing through the bottom avoiding opening 113, and the screw body 20 provided in the limiting cavity 111 abuts against the inner wall surface of the seat body 11 so that the taper portion 13 drills into the bone, to accordingly prevent the screw body 20 from dropping out of the bone.

The screw body 20 is detachably mounted on the fixed seat 10, in this way the screw body 20 passes through the top avoiding opening 112, the limiting cavity 111 and the bottom avoiding opening 113 of the seat body 11 in sequence when using the orthopedic implanted screw, and drills into the bone in a screwing mode. When the screw body 20 drills into the bone for a certain depth, the taper portion 13 of the fixed seat 10 may contact with a surface of the bone. At the moment, the screw body 20 is continued to be screwed. When the screw body 20 continues to drill into the bone, one end of the screw body 20 far away from the bone may abut against the deformation limiting portion 12 of the fixed seat 10, the deformation limiting portion 12 may be deformed until the one end of the screw body 20 far away from the bone completely gets over the deformation limiting portion 12, the deformation limiting portion 12 may restore an original shape to stop an end surface of the one end of the screw body 20 far away from the bone. Then the screw body 20 is continued to be screwed, and the screw body 20 positioned in the limiting cavity 111 abuts against the inner wall surface of the seat body 11 so that the taper portion 13 may drill into the bone. In this way, a limitation relation may be formed between the fixed seat 10 and the bone. Meanwhile, the fixed seat 10 further plays a role of limitation to the screw body 20, thereby preventing the screw body 20 from dropping out of the bone accordingly. Stability of the screw body 20 implanted into the bone is ensured, it is not only favorable to quick growth and stable growth and healing of the bone but prevents the affected area from secondary damage, and consequently living quality of the patient may be reliably improved.

As shown in Figs. 1-3 and Fig. 9, the screw body 20 may include a screw tip 21, a screw trunk 22 and a screw cap 23 connected in sequence. Herein, the screw tip 21 and the screw trunk 22 are provided with a threaded structure 24, or, the screw tip 21 is provided with a threaded structure 24,or, the screw trunk 22 is provided with a threaded structure 24,the screw tip 21 and the screw trunk 22 drill into the bone through the threaded structure 24, the screw cap 23 is limited in the limiting cavity 111 by the inner wall surface of the seat body 11 and the deformation limiting portion 12, and the screw cap 23 is configured to abut against the inner wall surface of the seat body 11. When the screw cap 23 abuts against the deformation limiting portion 12 of the fixed seat 10, the deformation limiting portion 12 may be deformed until the screw cap 23 completely gets over the deformation limiting portion 12 to enter into the limiting cavity 111, a part surface of the screw cap 23 may abut against the inner wall surface of the seat body 11, and the deformation limiting portion 12 may contact with an upper surface of the screw cap 23, as to limit the screw body 20 axially.

It is to be noted that the deformation limiting portion 12 is an elastic buckle with a deformation function. There are a plurality of elastic buckles, and the plurality of elastic buckles are provided around a circumferential direction of the top avoiding opening 112 at intervals. In this way, limiting stability of the deformation limiting portion 12 to the screw cap 23 may be improved.

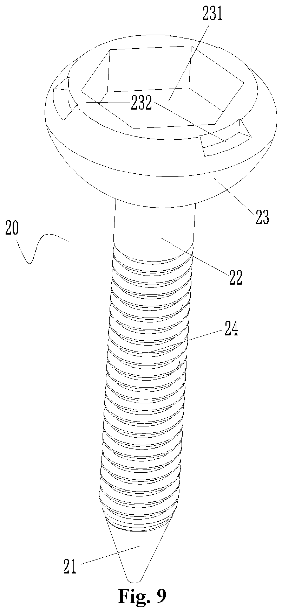

As shown in Fig. 9, the orthopedic implanted screw comprises a screwing counter bore 231 and a clamping slot 322 provided on an upper surface of the screw cap 23, The clamping slot 232 is connected with the deformation limiting portion 12 compatibly in a clamping mode. The screwing counter bore 231 is configured to facilitate screwing of the screw body 20, and the clamping slot 232 cooperates with the deformation limiting portion 12 correspondingly. When the screw cap 23 gets over the deformation limiting portion 12 to enter into the limiting cavity 111, the deformation limiting portion 12 may connect with the clamping slot 232 adaptably in a clamping mode. In this way, the screw body 20 is further stopped and limited to rotate.

In order to improve assembling stability of the screw body 20 and the seat body 11 and ensure that the seat body 11 provides the screw body 20 with enough support, optionally, a part surface of the inner wall surface of the seat body 11 and a part surface of an outer surface of the screw cap 23 are curved surfaces fitting with each other compatibly.

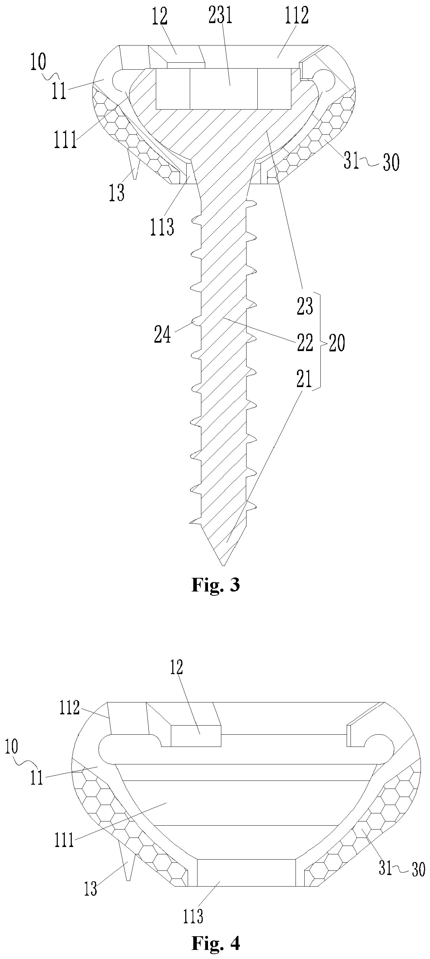

In the embodiment, as shown in Figs. 3-5 and Fig. 7, matching surfaces of the inner wall surface of the seat body 11 and the outer surface of the screw cap 23 compatibly are a part surface of a spherical surface. In this way, a greater contact area between the inner wall surface of the seat body 11 and the outer surface of the screw cap 23 may be ensured, and accordingly the seat body 11 may more effectively support and limit the screw body 20.

Optionally, in order to facilitate convenient integral manufacturing and formation of the taper portion 13 and the seat body 11, the taper portion 13 extends along an axial direction of the seat body 11.

Of course, in order to ensure that the seat body 11 and the bone would not get loose due to rotation of the screw body 20, improve rotation stopping and limitation functions of the seat body 11 to the screw body 20, optionally, the taper portion 13 inclines toward a side opposite to a direction in which the threaded structure 24 is rotated into an inside of the bone.

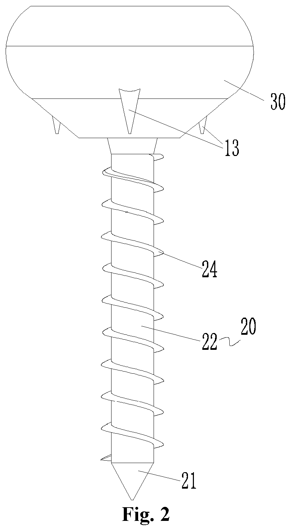

In order to improve connection stability of the seat body 11 and the bone, as shown in Fig. 2 and Figs. 5-6, there are a plurality of taper portions 13, and the plurality of taper portions 13 are provided around a circumferential direction of the seat body 11 at intervals.

As shown in Figs. 3-4 and Fig. 8, the orthopedic implanted screw further includes a pad 30. The pad 30 is provided on an outer wall surface of the fixed seat 10 In addition, the taper portion 13 passes through the pad 30, and an interior of the pad is provided with a plurality of micropore structures 31. The multiple micropore structures 31 may facilitate the bone to grow inward, thereby ensuring perfect combination of the orthopedic implanted screw and the bone accordingly.

In order to facilitate stabilization of an overall structure of the pad 30 and the fixed seat 10, the pad 30 and the fixed seat 10 are formed integrally through 3D printing.

It is to be noted that terms used here are to describe a specific embodiment only and not intended to limit the exemplary embodiment in accordance with the application. If it is used here, unless otherwise clearly specified in the context, a singular form should be intended to include a plural form. In addition, it is to be understood that when the term "include" and/or "contain" is used in the description, it indicates that there is a feature, a step, an operation, a device, a component and/or their combinations.

Unless otherwise specifically stated, a relative arrangement of the part and the step, a number expression and a value clarified in these embodiments may not limit the scope of the disclosure. Meanwhile, it is to be understood that, in order to facilitate description, a size of each part shown in the figure is not drawn in accordance with an actual proportional relation. A technology, a method and a device known by those of ordinary skilled in the related art may not be discussed in detail. However, the technology, the method and the device should be deemed as one part of an authorization description under appropriate conditions. In all examples shown and discussed here, any specific values should be explained to be exemplary merely rather than be restrictive. Therefore, other examples of the exemplary embodiments may have different values. It is to be noted that a similar reference number and a similar letter may represent similar terms in the figure below. Therefore, once defined in one figure, an item does not need to be further discussed in a subsequent figure.

In order to facilitate description, a spatial relative term may be used here, such as "over", "above", "on an upper surface" and "on", to describe a spatial position relationship between a device or a feature shown in the figure and other devices or other features. It is to be understood that the spatial relative term is intended to include different orientations of the device during use or operation outside the orientation described in the figure. For example, if the device in the figure is inverted, it may be described as that the device "above other devices or other structures" or "over other devices or other structures" shall be positioned "below other devices or other structures" or "under other devices or other structures". Therefore, an exemplary term "above" may include two orientations: "above" and "below". As an alternative, the device may be positioned with other different modes (90° rotation or positioned at other orientations), and the spatial relative description used here needs to be explained correspondingly.

It is to be noted that terms used here are to describe a specific embodiment only and not intended to limit the exemplary embodiment in accordance with the application. If it is used here, unless otherwise clearly specified in the context, a singular form should be intended to include a plural form. In addition, it is to be understood that when the term "include" and/or "comprise" is used in the description, it indicates that there is a feature, a step, working, a device, a component and/or their combinations.

It is to be noted that the terms "first", "second" and the like in the description, claims and the abovementioned drawings of the application are are used for distinguishing similar objects rather than describing a specific sequence or a precedence order. It is to be understood that data used in such a way may be exchanged under appropriate conditions, in order to facilitate implementation of the embodiment of the application described here in a sequence other than the one graphically shown or described here.

The above is only the preferred embodiment of the disclosure and not intended to limit the disclosure. For those skilled in the art, the disclosure may have various modifications and variations. The scope of protection of the disclosure is defined by the appended claims.

## Claims

1. An orthopedic implanted screw, for implanting into a bone, comprising:
a fixed seat (10), the fixed seat (10) comprising a seat body (11), a deformation limiting portion (12) and a taper portion (13), the seat body (11) is provided with a limiting cavity (111), a top avoiding opening (112) and a bottom avoiding opening (113) communicating with the limiting cavity (111), the deformation limiting portion (12) is provided on an inner wall surface of the seat body (11) and protrudes toward an inside of the limiting cavity (111), the deformation limiting portion (12) is provided at the top avoiding opening (112), and the taper portion (13) is convexly provided on an outer wall surface of the seat body (11); and
a screw body (20), the screw body (20) being detachably mounted on the fixed seat (10), the screw body (20) being configured to drill into the bone in a rotating mode, a part of the screw body (20) being limited in the limiting cavity (111) by the inner wall surface of the seat body (11) and the deformation limiting portion (12), another part of the screw body (20) drilling into the bone after passing through the bottom avoiding opening (113), and the screw body (20) provided in the limiting cavity (111) abutting against the inner wall surface of the seat body (11) so that the taper portion (13) drills into the bone, to accordingly prevent the screw body (20) from dropping out of the bone, **characterized in that**, the orthopedic implanted screw further comprises a pad (30), the pad (30) is provided on an outer wall surface of the fixed seat (10), the taper portion (13) passes through the pad (30), and an interior of the pad (30) is provided with a plurality of micropore structures (31).

2. The orthopedic implanted screw as claimed in claim 1, wherein, the screw body (20) comprises a screw tip (21), a screw trunk (22) and a screw cap (23) connected in sequence, wherein, the screw tip (21) and the screw trunk (22) are provided with a threaded structure (24),or, the screw tip (21) is provided with a threaded structure (24),or, the screw trunk (22) is provided with a threaded structure (24),the screw tip (21) and the screw trunk (22) drill into the bone through the threaded structure (24), the screw cap (23) is limited in the limiting cavity (111) by the inner wall surface of the seat body (11) and the deformation limiting portion (12), and the screw cap (23) is configured to abut against the inner wall surface of the seat body (11).

3. The orthopedic implanted screw as claimed in claim 2, wherein, a part surface of the inner wall surface of the seat body (11) and a part surface of an outer surface of the screw cap (23) are curved surfaces fitting with each other compatibly.

4. The orthopedic implanted screw as claimed in claim 2, wherein, matching surfaces of the inner wall surface of the seat body (11) and fitting the outer surface of the screw cap (23) are a part surface of a spherical surface.

5. The orthopedic implanted screw as claimed in claim 2, wherein,
the taper portion (13) extends along an axial direction of the seat body (11); or
the taper portion (13) inclines toward a side opposite to a direction in which the threaded structure (24) is rotated into an inside of the bone.

6. The orthopedic implanted screw as claimed in claim 5, wherein, there are a plurality of taper portions (13), and the plurality of taper portions (13) are provided around a circumferential direction of the seat body (11) at intervals.

7. The orthopedic implanted screw as claimed in claim 1, wherein, the deformation limiting portion (12) is an elastic buckle with adeformation function, there are a plurality of elastic buckles, and the plurality of elastic buckles are provided around a circumferential direction of the top avoiding opening (112) at intervals.

8. The orthopedic implanted screw as claimed in claim1, wherein, the pad (30) and the fixed seat (10) are formed integrally through 3D printing.

9. The orthopedic implanted screw as claimed in claim 2, wherein, the orthopedic implanted screw comprises a screwing counter bore (231) and a clamping slot (322) provided on an upper surface of the screw cap (23), and the clamping slot (232) is connected with the deformation limiting portion (12) compatibly in a clamping mode.

## Patentansprüche

1. Orthopädische Implantatschraube zum Implantieren in einen Knochen, mit:
einem fixierten Sitz (10), wobei der fixierte Sitz (10) einen Sitzkörper (11), einen Verformungsbegrenzungsabschnitt (12) und einen sich verjüngenden Abschnitt (13) aufweist, wobei der Sitzkörper (11) mit einem Begrenzungshohlraum (111), einer oberen Vermeidungsöffnung (112) und einer untere Vermeidungsöffnung (113) versehen ist, die mit dem Begrenzungshohlraum (111) in Verbindung stehen, wobei der Verformungsbegrenzungsabschnitt (12) an einer Innenwandfläche des Sitzkörpers (11) vorgesehen ist und in Richtung einer Innenseite des Begrenzungshohlraums (111) vorsteht, wobei der Verformungsbegrenzungsabschnitt (12) an der oberen Vermeidungsöffnung (112) vorgesehen ist und der sich verjüngende Abschnitt (13) an einer Außenwandfläche des Sitzkörpers (11) konvex vorgesehen ist; und
einem Schraubenkörper (20), wobei der Schraubenkörper (20) lösbar an dem fixierten Sitz (10) montiert ist, wobei der Schraubenkörper (20) eingerichtet ist, um in einem Drehmodus in den Knochen zu bohren, wobei ein Teil des Schraubenkörpers (20) in dem Begrenzungshohlraum (111) durch die Innenwandfläche des Sitzkörpers (11) und den Verformungsbegrenzungsabschnitt (12) begrenzt wird, wobei ein anderer Teil des Schraubenkörpers (20) nach einem Durchgehen durch die untere Vermeidungsöffnung (113) in den Knochen bohrt, und wobei der im Begrenzungshohlraum (111) vorgesehene Schraubenkörper (20) an der Innenwandfläche des Sitzkörpers (11) angrenzt, sodass der sich verjüngende Abschnitt (13) in den Knochen bohrt, um ein Herausfallen des Schraubenkörpers (20) aus dem Knochen zu verhindern,
**dadurch gekennzeichnet, dass** die orthopädische Implantatschraube des Weiteren ein Polster (30) umfasst, wobei das Polster (30) an einer Außenwandfläche des fixierten Sitzes (10) vorgesehen ist, der sich verjüngende Abschnitt (13) durch das Polster (30) hindurchgeht und ein Inneres des Polsters (30) mit einer Vielzahl von Mikroporenstrukturen (31) versehen ist.

2. Orthopädische Implantatschraube gemäß Anspruch 1, wobei der Schraubenkörper (20) eine Schraubenspitze (21), einen Schraubenschaft (22) und eine Schraubenkappe (23) aufweist, die nacheinander verbunden sind, wobei die Schraubenspitze (21) und der Schraubenschaft (22) mit einer Gewindestruktur (24) versehen sind, oder die Schraubenspitze (21) mit einer Gewindestruktur (24) versehen ist, oder der Schraubenschaft (22) mit einer Gewindestruktur versehen (24) ist, wobei die Schraubenspitze (21) und der Schraubenschaft (22) durch die Gewindestruktur (24) in den Knochen bohren, wobei die Schraubenkappe (23) in dem Begrenzungshohlraum (111) durch die Innenwandfläche des Sitzkörpers (11) und den Verformungsbegrenzungsabschnitt (12) begrenzt wird, und die Schraubenkappe (23) eingerichtet ist, um an der Innenwandfläche des Sitzkörpers (11) anzugrenzen.

3. Orthopädische Implantatschraube gemäß Anspruch 2, wobei eine Teilfläche der Innenwandfläche des Sitzkörpers (11) und eine Teilfläche einer Außenfläche der Schraubenkappe (23) gekrümmte Flächen sind, die aufeinander abgestimmt miteinander passen.

4. Orthopädische Implantatschraube gemäß Anspruch 2, wobei zusammenpassende Flächen der Innenwandoberfläche des Sitzkörpers (11), die mit der Außenfläche der Schraubenkappe (23) passen, eine Teilfläche einer kugelförmigen Fläche sind.

5. Orthopädische Implantatschraube gemäß Anspruch 2, wobei
sich der sich verjüngende Abschnitt (13) entlang einer Axialrichtung des Sitzkörpers (11) erstreckt; oder
der sich verjüngende Abschnitt (13) zu einer Seite geneigt ist, die einer Richtung entgegengesetzt ist, in der die Gewindestruktur (24) in ein Inneres des Knochens gedreht wird.

6. Orthopädische Implantatschraube gemäß Anspruch 5, wobei es mehrere sich verjüngende Abschnitte (13) gibt und die mehreren sich verjüngenden Abschnitte (13) um eine Umfangsrichtung des Sitzkörpers (11) herum in Abständen vorgesehen sind.

7. Orthopädische Implantatschraube gemäß Anspruch 1, wobei der Verformungsbegrenzungsabschnitt (12) ein elastischer Verschluss mit einer Verformungsfunktion ist, es mehrere elastische Verschlüsse gibt und die mehreren elastischen Verschlüsse um eine Umfangsrichtung der oberen Vermeidungsöffnung (112) herum in Abständen vorgesehen sind.

8. Orthopädische Implantatschraube gemäß Anspruch 1, wobei das Polster (30) und der fixierte Sitz (10) durch 3D-Druck als Einheit ausgebildet sind.

9. Orthopädische Implantatschraube gemäß Anspruch 2, wobei die orthopädische Implantatschraube eine Verschraubungssenkung (231) und eine Klemmlücke (322) umfasst, die an einer oberen Fläche der Schraubenkappe (23) vorgesehen sind, und wobei die Klemmlücke (232) in einem Klemmmodus aufeinander abgestimmt mit dem Verformungsbegrenzungsabschnitt (12) verbunden ist.

## Revendications

1. Vis implantée orthopédique, pour implantation dans un os, comprenant :
un siège fixé (10), le siège fixé (10) comprenant un corps de siège (11), une partie de limitation de déformation (12) et une partie effilée (13), le corps de siège (11) étant pourvu d'une cavité de limitation (111), d'une ouverture d'évitement supérieure (112) et d'une ouverture d'évitement inférieure (113) communiquant avec la cavité de limitation (111), la partie de limitation de déformation (12) étant disposée sur une surface de paroi interne du corps de siège (11) et faisant saillie vers l'intérieur de la cavité de limitation (111), la partie de limitation de déformation (12) étant prévu au niveau de l'ouverture d'évitement supérieure (112), et la partie effilée (13) étant prévue de manière convexe sur une surface de paroi externe du corps de siège (11) ; et
un corps de vis (20), le corps de vis (20) étant monté de manière amovible sur le siège fixé (10), le corps de vis (20) étant configuré pour pénétrer dans l'os de manière rotative, une partie du corps de vis (20) étant limitée dans la cavité de limitation (111) par la surface de paroi interne du corps de siège (11) et la partie de limitation de déformation (12), une autre partie du corps de vis (20) pénétrant dans l'os après être passée à travers l'ouverture d'évitement inférieure (113), et le corps de vis (20) prévu dans la cavité de limitation (111) venant en butée contre la surface de paroi interne du corps de siège (11) de sorte que la partie effilée (13) pénètre dans l'os, pour empêcher ainsi le corps de vis (20) de sortir de l'os, **caractérisée en ce que**, la vis implantée orthopédique comprend en outre un tampon (30), le tampon (30) est prévu sur une surface de paroi externe du siège fixé (10), la partie effilée (13) passe à travers le tampon (30), et un intérieur du tampon (30) est muni d'une pluralité de structures micropores (31).

2. Vis implantée orthopédique selon la revendication 1, dans laquelle le corps de vis (20) comprend une pointe de vis (21), un tronc de vis (22) et un chapeau de vis (23) reliés en séquence, dans laquelle la pointe de vis (21) et le tronc de vis (22) sont munis d'une structure filetée (24),ou, la pointe de vis (21) est munie d'une structure filetée (24),ou, le tronc de vis (22) est muni d'une structure filetée (24), la pointe de vis (21) et le tronc de vis (22) pénètre dans l'os au moyen de la structure filetée (24), le chapeau de vis (23) est limité dans la cavité de limitation (111) par la surface de paroi interne du corps de siège (11) et la partie de limitation de déformation (12), et le chapeau de vis (23) est configuré pour venir en butée contre la surface de paroi interne du corps de siège (11).

3. Vis implantée orthopédique selon la revendication 2, dans laquelle une surface partielle de la surface de paroi interne du corps de siège (11) et une surface partielle d'une surface externe du chapeau de vis (23) sont des surfaces incurvées s'adaptant l'une à l'autre de manière compatible.

4. Vis implantée orthopédique selon la revendication 2, dans laquelle un appariement de surfaces de la surface de paroi interne du corps de siège (11) et un ajustement de la surface externe du chapeau de vis (23) sont une surface partielle d'une surface sphérique.

5. Vis implantée orthopédique selon la revendication 2, dans laquelle
la partie effilée (13) s'étend le long d'une direction axiale du corps de siège (11) ; ou
la partie effilée (13) s'incline vers un côté opposé à une direction dans laquelle la structure filetée (24) est tournée dans un intérieur de l'os.

6. Vis implantée orthopédique selon la revendication 5, dans laquelle il y a une pluralité de parties effilées (13), et la pluralité de parties effilées (13) sont prévues autour d'une direction circonférentielle du corps de siège (11) à des intervalles.

7. Vis implantée orthopédique selon la revendication 1, dans laquelle la partie de limitation de déformation (12) est une boucle élastique avec une fonction de déformation, il y a une pluralité de boucles élastiques, et la pluralité de boucles élastiques sont prévues autour d'une direction circonférentielle de l'ouverture d'évitement supérieure (112) à des intervalles.

8. Vis implantée orthopédique selon la revendication 1, dans laquelle le tampon (30) et le siège fixé (10) sont formés d'un seul tenant par impression 3D.

9. Vis implantée orthopédique selon la revendication 2, dans laquelle la vis implantée orthopédique comprend un contre-alésage de vissage (231) et une fente de serrage (322) prévue sur une surface supérieure du chapeau de vis (23), et la fente de serrage (232) est reliée à la partie de limitation de déformation (12) de manière compatible dans un mode de serrage.
